# EUROPÄISCHE PATENTANMELDUNG

(11) **EP 1 923 807 A2**
(43) Veröffentlichungstag der Anmeldung: **21.05.2008**
(21) Anmeldenummer: 07022188.2
(22) Anmeldetag: 15.11.2007
(51) Int. Cl.: G06F 19/00

(54) **Verfahren sowie System zur anwendungsbezogenen Bereitstellung von Patienteninformationen**

(30) Priorität: 15.11.2006 DE 102006054194
(71) Anmelder: Optiplan Gesellschaft für optische Planungsgeräte mit beschränkter Haftung, 40489 Düsseldorf (DE)
(72) Erfinder: Wagner, Herbert, 40489 Düsseldorf (DE); Lutz, Guido, 47279 Duisburg (DE)
(74) Vertreter: Stenger, Watzke & Ring

(57) **Zusammenfassung**

Die Erfindung betrifft ein Verfahren zur anwendungsbezogenen Bereitstellung von Patienteninformationen. Um ein Verfahren zur Verarbeitung von Patienteninformationen bereitzustellen, welches eine übersichtliche Organisation und eine einfache Zugänglichkeit bietet, wird mit der Erfindung ein Verfahren vorgeschlagen, bei dem Patienteninformationen nach vorgebbaren Kriterien erfaßt und in einer Datenbank (4) gespeichert werden, bei dem die in der Datenbank (4) gespeicherten Patienteninformationen anwendungsbezogen zusammengestellt und angezeigt werden, wobei für eine anwendungsbezogene Anzeige von Patienteninformationen fachliche Kriterien berücksichtigende Formulare (6) hierarchieunabhängig verwendet werden, denen Patienteninformationen formularabhängig zugeordnet werden.

## Beschreibung

Die Erfindung betrifft ein Verfahren zur anwendungsbezogenen Bereitstellung von Patienteninformationen. Darüber hinaus wird mit der Erfindung ein entsprechendes System zur anwendungsbezogenen Bereitstellung von Patienteninformationen vorgeschlagen.

Bisher wird die Pflegedokumentation beispielsweise in Altenheimen handschriftlich von behandelnden Pflegern und Ärzten in Patientenakten eingetragen, wobei die Patientenakten eine Vielzahl von Patienteninformationen aufweisenden Formularen enthalten. Zu den Patienteninformationen gehören beispielsweise persönliche Daten und Daten bezüglich Ansprechpartner, Betreuer, behandelnde Ärzte, externe Dienstleister, ehrenamtliche Hilfen, zuständige Kirchengemeinden, benötigte und mitgebrachte Hilfsmittel bis hin zu medizinischen Fakten. Zudem werden Informationen über vorhandene Ressourcen erfaßt, die beispielsweise Therapieangebote, Unterhaltungs- und Freizeitaktivitäten und ähnliches betreffen. Aus den patientenbezogenen und den ressourcenbezogenen Informationen läßt sich ein sorgfältiger Plan erstellen, nach dem der Patient einen möglichst angenehmen Heimalltag in Form eines optimal auf den Patienten zugeschnittenen Tagesablaufs erhält. Ändern sich patienten- oder ressourcenbezogene Informationen, wird ein an die neuen Bedingungen angepaßter Plan erstellt, der den Änderungen Rechnung trägt. Durch dieses Pflegedokumentationssystem entsteht eine große Anzahl von Daten und Informationen bezüglich der individuell gestalteten Pflege eines Patienten, die allen an der Pflege und Betreuung beteiligten Personen zur Verfügung gestellt werden sollten. Dazu gehören beispielsweise pflegende Mitarbeiter, alle Mediziner, alle Therapeuten und alle ehrenamtlichen Mitarbeiter, die alle einen einfachen und zügigen Zugriff auf die für sie wichtigen Patienteninformationen haben sollten. Da aber Formulare wie Stammblatt, Pflegeanamnese, Pflegeplanung, Pflegebericht/Evaluation, Qualitätskontrolle, Pflegevisite, Leistungsnachweis Pflege, Leistungsnachweis medizinische Behandlung, Leistungsnachweis Therapie, ärztliches Verordnungsblatt, Vitalwerte, Medikamentenblatt, Medical Bedarf, Bewegungsplan, Braden-Skala, Dokumentation des Sturtzrisikos, Sturtzprotokoll, Wundmanagement, freiheitsentziehende Maßnahmen, Handzeichenliste und Überleitungsbogen vorgesehen sein können, ist das Herausfiltern von speziellen benötigten Patienteninformationen sehr zeit- und arbeitsaufwendig und zudem unübersichtlich.

Daher ist es die **Aufgabe** der vorliegenden Erfindung, ein Verfahren zur Verarbeitung von Patienteninformationen bereitzustellen, welches eine übersichtliche Organisation und einfache Zugänglichkeit bietet. Insbesondere sollen in den Pflegebereichen bekannte und eingesetzte Formulare in einer automatisierten Form einsetzbar sein.

Zur **Lösung** dieser Aufgabe wird mit der Erfindung ein Verfahren zur anwendungsbezogenen Bereitstellung von Patienteninformationen vorgeschlagen, bei dem Patienteninformationen nach vorgebbaren Kriterien erfaßt und in einer Datenbank gespeichert werden, bei dem die in der Datenbank gespeicherten Patienteninformationen anwendungsbezogen zusammengestellt und angezeigt werden, wobei für eine anwendungsbezogene Anzeige von Patienteninformationen fachliche Kriterien berücksichtigende Formulare hierarchieunabhängig verwendet werden, die den Patienteninformationen formularabhängig zugeordnet werden.

Mit der Erfindung wird vorgeschlagen, daß die Patienteninformationen in Datenbanken gespeichert werden. Die Erfassung der Patienteninformationen erfolgt beispielsweise durch manuelles Eintragen der Patienteninformationen in dafür vorgesehene elektronische Formulare, die von einer Anzeigeeinheit, beispielsweise von einem Monitor eines Rechners angezeigt werden. Diese Formulare entsprechen vorzugsweise äußerlich und inhaltlich den bisher bekannten, händisch zu verwendenden Formularen, wodurch die an der Pflege eines Patienten beteiligten Personen nicht zur Anwendung des Verfahrens angelernt werden müssen bzw. sich in gewohnter Umgebung bewegen. Je nach Aufgabengebiet der die Informationen eingebenden Person werden die Patienteninformationen in fachliche Kriterien berücksichtigende Formulare eingetragen. Die in den Datenbanken gespeicherten Patienteninformationen werden dann zur Darstellung den Formularen zugeordnet und unter Berücksichtigung von fachlichen Kriterien dargestellt. So können beispielsweise einem Therapeuten die für ihn wichtige Informationen angezeigt werden, ohne daß der Therapeut selbst diese Informationen zusammenstellen muß. Dadurch ist ein schneller Zugriff und eine übersichtliche Darstellung von Patienteninformationen gegeben, was zudem die Sicherheit der Pflege eines Patienten erhöht.

Durch die beschriebene elektronische Datenverarbeitung werden die komplexen Abläufe beispielsweise der Pflegeplanung in einzelne Sequenzen unterteilt, wodurch es ermöglicht ist, die Abläufe aus den unterschiedlichsten Blickwinkeln zu betrachten.

Die elektronischen Formulare sind übersichtlich gestaltet, so daß ihnen Informationen einfach und schnell entnommen werden können. Es ist auch eine einfache und schnelle Informationseingabe möglich. Verschiedene Bereiche der Formulare können beispielsweise farblich unterlegt sein, so daß mit den Farben eine spezielle Information assoziiert werden kann, ohne daß nach bestimmten Informationen inhaltlich gesucht werden muß. Auch diese Ausgestaltung vereinfacht die Handhabung des elektronischen Pflegedokumentationssystems. Vorzugsweise ist eine spezielle Pflegedokumentationssoftware auf allen in einem Pflegeheim oder Krankenhaus befindlichen Rechnern vorhanden, wobei diese Rechner zudem vernetzt sein können, so daß ein Zugriff auf das System von allen angeschlossenen Rechnern möglich ist. Dieses erübrigt das Herumtragen von Akten, welche immer nur einer Person zur Verfügung stehen. Zudem werden keine Aktenschränke benötigt, welche bei einer großen Anzahl an Patienten viel Platz benötigen.

Erfindungsgemäß ist verfahrensseitig vorgesehen, Patientendaten zunächst einmal zu erfassen, was beispielsweise mittels elektronischer Formulare erfolgen kann. Dabei erfolgt die Erfassung der Patienteninformationen nicht willkürlich, sondern nach vorgebbaren Kriterien. Derlei Kriterien können sich beispielsweise aufgrund von Erfahrungswerten der das erfindungsgemäße Verfahren anwendenden Person ergeben. Sie dienen einerseits dazu, eine vollständige und vorzugsweise fehlerfreie Aufnahme von Patienteninformationen zu ermöglichen. Andererseits können vorgebbare Kriterien dazu genutzt werden, individuellen Erfassungswünschen gerecht zu werden. Das erfindungsgemäße Verfahren kann beispielsweise zur Altenpflege in Altenheimen genutzt werden. Dementsprechend können von Altenheim zu Altenheim unterschiedliche Anforderungen zur Erfassung von Patienteninformationen vorgesehen sein. Mittels der vorgebbaren Kriterien zur Erfassung von Patienteninformationen kann dies berücksichtigt werden.

Die erfaßten Patienteninformationen werden in einer Datenbank gespeichert. Sie werden so für eine Abfrage verfügbar gehalten. Dabei ist eine mögliche Abfrage der gespeicherten Informationen in vorteilhafter Weise völlig unabhängig von der zuvor durchgeführten Informationserfassung. Nach dem erfindungsgemäßen Verfahren werden die in der Datenbank gespeicherten Patienteninformationen vielmehr anwendungsbezogen zusammengestellt und angezeigt. Zu diesem Zweck ist vorgesehen, daß fachliche Kriterien berücksichtigende Formulare verwendet werden, denen Patienteninformationen formularabhängig zugeordnet werden. Dabei sind die Formulare hierarchieunabhängig organisiert, so daß in einfacher und schneller Weise ein Zugriff auf anwendungsbezogene Informationen möglich ist. Anders als bisher ist es insofern nicht mehr erforderlich, sich durch eine Hierarchie von Informationsformularen zu bewegen. Anwendungsbezogene Patienteninformationen werden entsprechenden Formularen vielmehr hierarchieunabhängig zugeordnet, was bediener- bzw. verwenderseitig einen sicheren und schnellen Zugriff erlaubt.

Bei der anwendungsbezogenen Bereitstellung von Patienteninformationen handelt es sich um die Bereitstellung von aufbereiteten Informationen, das heißt von solchen Informationen, die bestimmten vorgebbaren Auswahlkriterien gerecht werden. So kann beispielsweise als eine anwendungsbezogene Bereitstellung von Patienteninformationen vorgesehen sein, all die Informationen zusammenzufassen und aufzubereiten, die zum Themenbereich Medikation gehören. Gemäß einer solchen anwendungsbezogenen Bereitstellung von Patienteninformationen würden dann all diejenigen Informationen zur Darstellung gebracht werden, die den Themenkomplex der Medikation zuzurechnen sind, wie zum Beispiel die Auflistung der von einem Patienten regelmäßig eingenommenen Medikamente, Nebenwirkungen dieser Medikamente, Grund der Medikamentenverordnung und dergleichen mehr. Diese in der Datenbank gespeicherten Informationen können auf ganz unterschiedlichem Wege, das heißt aufgrund von räumlich und/oder zeitlich voneinander getrennten Informationserfassungen von der Datenbank aufgenommen und in dieser gespeichert sein. Das erfindungsgemäße Verfahren erlaubt gleichwohl eine Zusammenstellung dieser Informationen, und zwar anwendungsbezogen, so daß in schneller und zuverlässiger Weise eine konzentrierte Informationsvermittlung möglich ist.

Die zur Darstellung von anwendungsbezogenen Patienteninformationen genutzten Formulare können bediener- bzw. verwenderseitig selbst erstellt werden, so daß eine auf individuelle Bedürfnisse zugeschnittene Informationserfassung und/oder Darstellung möglich ist. Sowohl bei der Erfassung als auch der Darstellung von Patienteninformationen kommen bevorzugterweise fachliche Kriterien berücksichtigende Formulare zum Einsatz. Unter "fachliche Kriterien" sind dabei solche Kriterien zu verstehen, die zum jeweiligen Anwendungsfall fachlichen Bezug haben.

Gemäß einem weiteren Merkmal der Erfindung ist vorgesehen, daß auch für eine Erfassung von Patienteninformationen fachliche Kriterien berücksichtigende Formulare verwendet werden. Eine effiziente und zuverlässige Informationserfassung wird auf diese Weise sichergestellt. Dabei kann für eine weitere Vereinfachung des gesamten Verfahrens vorgesehen sein, daß ein auf den jeweiligen Anwendungsfall bezogenes Formular verwendet wird, mit dem Informationen sowohl erfaßt als auch angezeigt werden. Die Anzahl der zum Einsatz kommenden Formulare kann so minimiert werden, was die Verwendbarkeit, das heißt die Anwendung des erfindungsgemäßen Verfahrens vereinfacht. In diesem Zusammenhang kann beispielsweise vorgesehen sein, daß mit einem Formular bereits erfaßte und aufbereitete Daten, das heißt Patienteninformationen angezeigt werden, wobei die Bedienperson die Möglichkeit hat, mit Hilfe des bereits genutzten Formulars den bereits schon im System vorhandenen Informationen weitere beizufügen, das heißt eine Informationserfassung unter Verwendung eines anwendungsbezogenen Formulars durchzuführen. Insbesondere die nachträgliche Erfassung von Patienteninformationen wird so vereinfacht, weil das zu diesem Zweck zum Einsatz kommende anwendungsbezogene Formular eine Übersicht über unter Umständen noch fehlende Informationen in einfacher Weise zur Verfügung stellt.

Es kann in diesem Zusammenhang gemäß einem weiteren Merkmal der Erfindung auch vorgesehen sein, daß bestimmte von einem anwendungsbezogenen Formular bereitgestellte Informationen bediener- bzw. verwenderseitig nicht geändert werden können. Dies macht beispielsweise bei sogenannten Stammdaten, das heißt sogenannten Stamminformationen Sinn. Zu den Stammdaten bzw. Stamminformationen eines Patienten gehören beispielsweise patientenbezogene unveränderliche Informationen, wie zum Beispiel der Name eines Patienten, dessen Alter oder dergleichen. Diese Patienteninformationen werden zu Beginn aufgenommen und - falls erforderlich und/oder sinnvoll - in einem anwendungsbezogenen Formular zur Ansicht gebracht. Diese in dem Formular angezeigten Stammdaten können jedoch nicht verändert werden, so daß in jedem Fall eine weitreichende und auch auf andere Anwendungsformulare durchschlagende Informationsänderung vermieden, zumindest aber nicht ohne besondere Autorisierung ermöglicht ist. Informationfehleingaben können so in vorteilhafter Weise auf ein Minimum reduziert werden.

Gemäß einem weiteren Merkmal der Erfindung ist vorgesehen, daß Patienteninformationen nach ihrer Erfassung einer Prüfung unterzogen werden. Auch hierdurch wird die Erfassung von Fehlinformationen minimiert. Bevorzugterweise wird eine Prüfung auf Plausibilität durchgeführt. Eine solche Prüfung erfordert keine zusätzliche Fachkenntnis seitens des Bedieners und/oder Verwenders. Die Prüfung wird systemautark durchgeführt, wobei überprüft wird, ob die erfaßten Informationen dem Grunde nach überhaupt richtig sein können. Wenn beispielsweise dem System einerseits die Information mitgeteilt wird, daß ein bestimmter Patient seit über zehn Jahren ein bestimmtes Medikament zu sich nimmt, dieses Medikament aber erst seit fünf Jahren überhaupt am Markt erhältlich ist, ergibt sich hieraus eine mangelnde Plausibilität, was dem Bediener bzw. Verwender zwecks Überprüfung der miteinander inhaltlich kollidierenden Informationen angezeigt wird.

Gemäß einem weiteren Merkmal der Erfindung ist vorgesehen, daß die Erfassung von bestimmten Informationen zur automatischen Generierung weiterer Informationen genutzt wird. So kann beispielsweise die Erfassung der Information, daß ein bestimmter Patient ein bestimmtes Medikament erhält, dazu führen, daß ein automatischer Eintrag in die Datenbank generiert wird, wonach beispielsweise bestimmte andere, mit dem genommenen Medikament nicht zu applizierende Medikamente aufgelistet und beispielsweise in Form eines Warnhinweises aufbereitet zur Verfügung gestellt werden. Einer Fehlmedikation kann so besser vorgebeugt werden, weil das erfindungsgemäße Verfahren automatisch dafür sorgt, daß nicht nur das verschriebene Medikament sondern auch alle in Kombination mit diesem Medikament nicht einzunehmende Medikamente angezeigt werden. Die Pflege von Patienten kann mit Hilfe des erfindungsgemäßen Verfahrens insofern deutlich verbessert werden.

Gemäß einem weiteren Merkmal der Erfindung wird in diesem Zusammenhang vorgeschlagen, daß die weitere Information in Abhängigkeit einer bereits erfaßten Information aus einer vorgebbaren Liste automatisch ausgewählt und für eine Darstellung aufbereitet wird. Diese erfindungsgemäße Ausgestaltung hat den Vorteil, daß das gesamte Vermittlungssystem fachspezifisch ergänzt werden kann, insofern also lernfähig ist. Die Listen, aus denen sich ergänzende Informationen automatisch generieren, können fortlaufend erweitert und ausgebaut werden, was zugunsten der Patienten zu einer erheblichen Sicherheitsverbesserung führt.

Mit der Erfindung wird im übrigen ein System zur anwendungsbezogenen Bereitstellung von Patienteninformationen vorgeschlagen, das über elektronische und nicht elektronische Formulare zur Erfassung von Patienteninformationen verfügt, wobei die nicht elektronischen Formulare für eine automatisierte Informationsübertragung in die elektronischen Formulare eindeutig zuordbare Informationsfelder aufweisen.

Im alltäglichen Praxiseinsatz erfolgt die Informationserfassung in aller Regel noch über handschriftlich auszufüllende Formulare. Die auf diese Weise erfaßten Informationen müssen für eine erfindungsgemäße Verfahrensabwicklung ins Informationssystem übernommen, das heißt in die Datenbank eingespeist werden. Diese Übertragung kann manuell erfolgen, was aber zu Eingabefehlern führen kann. Um hier vorzubeugen, wird mit dem erfindungsgemäßen System vorgeschlagen, daß eine Informationsübertragung in die Datenbank in automatischer Weise erfolgt. Zu diesem Zweck werden erfindungsgemäß nicht elektronische Formulare einerseits und elektronische Formulare andererseits genutzt, die in ihrem Aufbau völlig identisch sein können. In jedem Fall verfügen die nicht elektronischen Formulare über eineindeutig zuordbare Informationsfelder, was es ermöglicht, diese in automatisierter Weise zu erkennen, die darin abgelegte Information zu erfassen und zur Speicherung in der Datenbank zu digitalisieren. Im einfachsten Fall kann beispielsweise vorgesehen sein, daß ein handschriftlich ausgefülltes, nicht elektronisches Formular eingescannt wird. Die eingescannte Information wird digitalisiert und aufgrund der eineindeutigen Zuordbarkeit von Informationsfeldern in richtiger Weise in die elektronische Form übernommen.

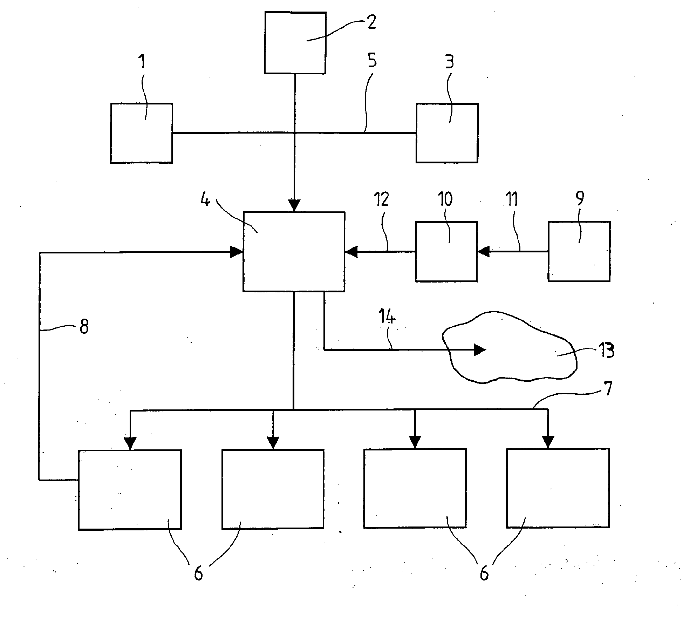

Weitere Merkmale und Vorteile der Erfindung ergeben sich aus der nachfolgenden Beschreibung anhand der einzigen Fig. nach Fig. 1, die in schematischer Darstellung eine erfindungsgemäße Verfahrensabwicklung zeigt.

Nach der erfindungsgemäßen Verfahrensabwicklung ist vorgesehen, daß Patienteninformationen nach vorgebbaren Kriterien erfaßt und in einer Datenbank 4 gespeichert werden. Dabei kann die Datenerfassung auf unterschiedliche Weise erfolgen. So kann beispielsweise vorgesehen sein, eine erste Informationserfassung durch eine direkte Befragung des Patienten 1 zu erreichen. Gegebenenfalls können auch Angehörige 2 des Patienten 1 befragt werden. Schließlich können Dritte 3 noch über Informationen verfügen. Dritte 3 können beispielsweise Krankenhauseinrichtungen, Altenheime, Ärzte oder sonstige Einrichtungen sein. Die von diesen in Fig. 1 exemplarisch dargestellten Personen und/oder Einrichtungen 1, 2 und 3 zur Verfügung gestellten Patienteninformationen werden in Entsprechung des Pfeils 5 in die Datenbank 4 eingespeist und dort gespeichert. Die so von der Datenbank 4 erfaßten Informationen können nun anwendungsbezogen bereitgestellt werden. Hierzu dienen elektronische Formulare 6, denen die Datenbank 4 anwendungsbezogen Informationen zuordnet, die dann zur Anzeige gebracht werden können, wie der Pfeil 7 symbolisiert.

Dabei kann gemäß einer Verfahrensvariante vorgesehen sein, daß die elektronischen Formulare 6 nicht nur der Darstellung von Informationen dienen, sondern selbst auch zur Informationserfassung genutzt werden können. Die Informationserfassung mittels eines Formulars 6 ist durch den Pfeil 8 symbolisiert.

Eine Informationserfassung kann gemäß einer Option der Erfindung auch mittels eines nicht elektronischen Formulars 9 erfolgen. Die von einem solchen Formular 9 zur Verfügung gestellten Informationen werden mittels beispielsweise eines Scanners erfaßt und digitalisiert. Zu diesem Zweck wird das Formular 9 in eine entsprechende Scanneinheit gegeben, was durch den Pfeil 11 symbolisiert ist. Die vom Scanner 10 erfaßten Daten gelangen dann in Entsprechung des Pfeils 12 zur Speicherung in die Datenbank 4.

Es kann gemäß einem weiteren Merkmal des beschriebenen Ausführungsbeispiels vorgesehen sein, daß die Datenbank 4 über eine kommunikationstechnische Verbindung mit einem übergeordneten Kommunikationsnetz, beispielsweise dem Internet 13 verbunden ist. Auf diese Weise kann eine Kommunikation zwischen der in der Fig. dargestellten Datenbank 4 und einer anderen und über weitere Informationen verfügende Datenbank vorgenommen werden, so daß ein Informationsaustausch möglich ist. Ein solcher Informationsaustausch kann beispielsweise dann sehr sinnvoll sein, wenn ein Patient aus einem Altenheim in ein Krankenhaus verlegt wird. Die patientenbezogenen Daten können dann in einfacher Weise zwischen dem Altenheim und dem Krankenhaus ausgetauscht werden, und zwar ohne daß die Gefahr einer Fehlinformation besteht. Sämtliche sich um einen Patienten kümmernde Personen sind dann dank des erfindungsgemäßen Verfahrens vollständig über den Patienten und dessen Kranken- und/oder Pflegehistorie informiert.

### Bezugszeichenliste

- 1: Patient
- 2: Angehörige
- 3: Dritter
- 4: Datenbank
- 5: Pfeil
- 6: elektronisches Formular
- 7: Pfeil
- 8: Pfeil
- 9: nicht elektronisches Formular
- 10: Scanner
- 11: Pfeil
- 12: Pfeil
- 13: Internet
- 14: kommunikationstechnische Verbindung

## Patentansprüche

1. Verfahren zur anwendungsbezogenen Bereitstellung von Patienteninformationen, bei dem Patienteninformationen nach vorgebbaren Kriterien erfaßt und in einer Datenbank (4) gespeichert werden, bei dem die in der Datenbank (4) gespeicherten Patienteninformationen anwendungsbezogen zusammengestellt und angezeigt werden, wobei für eine anwendungsbezogene Anzeige von Patienteninformationen fachliche Kriterien berücksichtigende Formulare (6) hierarchieunabhängig verwendet werden, denen Patienteninformationen formularabhängig zugeordnet werden.

2. Verfahren nach Anspruch 1, **dadurch gekennzeichnet, daß** auch für eine Erfassung von Patienteninformationen fachliche Kriterien berücksichtigende Formulare (6) verwendet werden.

3. Verfahren nach Anspruch 1 oder 2, **dadurch gekennzeichnet, daß** ein auf den jeweiligen Anwendungsfall bezogenes Formular (6) verwendet wird, mit dem Informationen sowohl erfaßt als auch angezeigt werden.

4. Verfahren nach einem der vorhergehenden Ansprüche, **dadurch gekennzeichnet, daß** Patienteninformationen nach ihrer Eingabe einer Prüfung unterzogen werden.

5. Verfahren nach einem der vorhergehenden Ansprüche, **dadurch gekennzeichnet, daß** die Erfassung von bestimmten Informationen zur automatischen Generierung weiterer Informationen genutzt wird.

6. Verfahren nach Anspruch 5, **dadurch gekennzeichnet, daß** weitere Informationen in Abhängigkeit von erfaßten Informationen aus einer vorgebbaren Liste automatisch ausgewählt und für eine Darstellung aufbereitet werden.

7. System zur anwendungsbezogenen Bereitstellung von Patienteninformationen, mit elektronischen und nicht elektronischen Formularen (6, 9) zur Erfassung von Patienteninformationen, wobei die nicht elektronischen Formulare (9) für eine automatisierte Informationsübertragung in die elektrischen Formulare (6) eindeutig zuordbare Informationsfelder aufweisen.
